# EUROPEAN PATENT APPLICATION

(11) **EP 2 481 395 A1**
(43) Date of publication of application: **01.08.2012**
(21) Application number: 11152894.9
(22) Date of filing: 01.02.2011
(51) Int. Cl.: A61K 9/00, A61K 9/14, A61K 9/16, A61K 31/245

(54) **Sachet, effervescent tablet and dry syrup of otilonium**

(71) Applicant: Deva Holding Anonim Sirketi, Kucukcekmece/Istanbul (TR)
(72) Inventor: Haas, Philipp Daniel, 34303, ISTANBUL (TR); Sivasligil, Ramazan, 34303, ISTANBUL (TR); Koc, Fikret, 34303, ISTANBUL (TR); Kandemir, Levent, 34303, ISTANBUL (TR)

(57) **Abstract**

This invention is related to sachet, effervescent, dry syrup formulations of otilonium or pharmaceutically acceptable salts and as well as processes of preparation of these dosage forms.

## Description

### Technical Field

This invention is related to sachet, effervescent, dry syrup formulations of otilonium or pharmaceutically acceptable salts and as well as processes of preparation of these dosage forms.

### Background Art

Irritable Bowel Syndrome is a highly prevalent functional gastrointestinal disorder affecting up to 3-15% of the general population in Western Countries. Irritable bowel syndrome is a disorder characterized most commonly by cramping, abdominal pain, bloating, constipation, and diarrhea. Although there is no cure for IBS, there are treatments and medications which attempt to relieve symptoms.

Antispasmodics are commonly prescribed, which help to control colon muscle spasms and reduce abdominal pain. The antispasmodic otilonium bromide (OB) is used world-wide for the treatment of irritable bowel syndrome (IBS).

Otilonium bromide is a quaternary ammonium salt characterized by a long aliphatic chain. The chemical name for otilonium bromide is diethylmethyl {[(octyloxy-2 benzamido)-4 benzoyloxy] 2-ethyl} ammonium bromide.

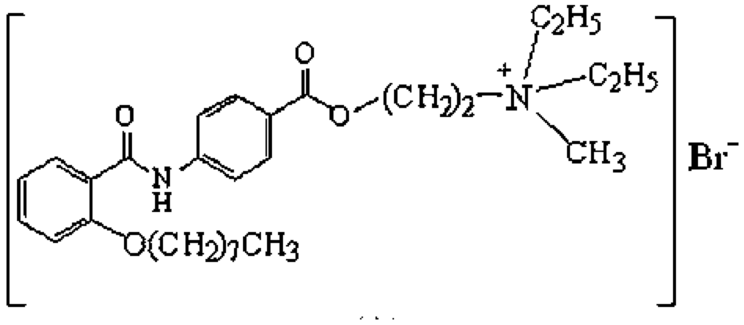

Its chemical properties are responsible for both a poor penetration in central nervous system and a prolonged binding to cell membranes.

Otilonium bromide reduces the frequency of abdominal pain and associated the severity of abdominal bloating by modifying Ca++ fluxes from extra and intracellular sites.

Pharmacokinetic studies have shown that following to oral administration, otilonium bromide has poor systemic absorption and exerts its activity principally on distal gastrointestinal tract. Otilonium bromide accumulates in the lower intestine and its spasmolytic effect has a direct action on the contractile proteins of the smooth muscle.

SUTTON, et al. The clinical pharmacology of single doses of otilonium bromide in healty volunteers. European Journal of Clinical Pharmacology 1997, no.52, p.365-369. discloses that when orally administered at doses that produce spasmolytic effects in humans, otilonium bromide was devoid of both central and peripheral atropine-like side effects.

Spasmoctyl® includes otilonium bromide which is on the market as a reference drug for oral solid dosage form.

Otilonium bromide can be used in formulations those differ significantly in their ways of administration such as oral tablets and topical application.

EP 0270503 B (A.MENARINI INDUSTRIE FARMACEUTICHE RI UNITE S.R.L) 15.09.1993 discloses a new pharmaceutical form of otilonium bromide which is suitable to effect a local application in the gastrointestinal tract, i.e. a topical direct administration, rather than an oral administration.

### Summary of invention

This invention is related to sachet, effervescent, dry syrup formulations of otilonium or pharmaceutically acceptable salts and as well as processes of preparation of these dosage forms.

### Technical Problem

An important percentage of patients suffering from irritable bowel syndrome are people showing a relatively high incidence of dysphagia. Such people may be unable to perform the necessary reflex or may be feeling sick or unwilling and/or unable to swallow tablets, capsules or other traditional oral solid dosage forms.

Anyone can have a swallowing disorder, but it is more likely in the high risk patients especially, the elderly people, stroke and rehabilitation patients and patients with malnutrition, neurodegenerative disease, pneumonia, or heart disease and children.

Likewise pills are not always acceptable to children and indeed many adults find them objectionable to take. Results of a US nationwide survey reveal that a large percentage (40%) of American adults has experienced difficulty swallowing pills, even though most have had no problems swallowing food or liquid.

**YAN ZHAO, LI DING,** . Determination of the unstable drug otilonium bromide in human plasma by LC-ESI-MS and its application to a pharmacokinetic study. Journal of Chromatography. 2010, vol.B, no.878, p.2896-2900. discloses that the stability tests indicate that otilonium bromide degrades rapidly in plasma and readily undergoes hydrolysis by the plasma esterase to yield two degradation products named DP-1 and DP-2.

DP-1:

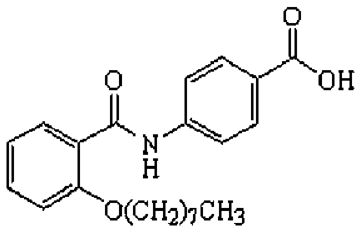

And DP-2 :

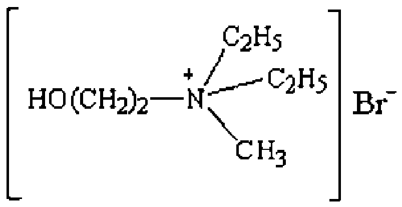

### Solution to Problem

In the present invention, inventors provide alternative dosage forms and pharmaceutical formulations to conventional oral solid dosage forms of otilonium or pharmaceutically acceptable salts thereof.

In order to enhance the patient compliance in the treatment of irritable bowel syndrome, the compositions of this invention are conveniently presented in the suitable form of sachets, effervescent formulations and dry syrups for IBS patients. Such forms of otilonium or pharmaceutically acceptable salts need to be mixed with water before use and may be administered as fully dissolved, dispersed or suspended in a solution.

For IBS patients, oral administration of the powder or tablet in solution or dispersion in particular provides several advantages over conventional oral solid dosage forms. As the drug product is already in solution at the time it is consumed, reduced localized contact of the drug in the upper gastrointestinal tract leads to less irritation and greater tolerability to IBS patients. Buffered drug solution prevents gastric acids from interacting with the active pharmaceutical ingredient, which can be a major cause of stomach and esophageal upsets.

Since the drug is administered as a solution, problems associated with disintegration and dissolution of oral solid dosage forms are avoided as well.

Advantages of presented pharmaceutical formulations of otilonium or pharmaceutically acceptable salts compared with other oral dosage forms include an opportunity for formulator to improve taste. Superior taste masking is achieved by complementing formulations with flavours and/or sweeteners.

IBS patients make use of unit dose powders and effervescent tablets which are less risky of misuse, practical to carry and can be used in the case of sickness.

**YAN ZHAO, LI DING,** . Determination of the unstable drug otilonium bromide in human plasma by LC-ESI-MS and its application to a pharmacokinetic study. Journal of Chromatography B. 2010, vol.B, no.878, p.2896-2900. discloses that drugs like otilonium bromide that are known to be unstable when stored in an aqueous solution. It is concluded that otilonium or pharmaceutically acceptable salt should not be contacted with water for a time of period to satisfy the stability before usage. Effervescent, sachet or dry syrup of otilonium bromide provides a non-aqueous environment of active pharmaceutical ingredient during the storage. When they contact with water or saliva at using stage of patients, it does not bring about degradations.

### Description of embodiments

In the first aspect of this invention, inventors provide a pharmaceutical formulation and a manufacturing process for the preparation of the unit dose powder formulation of otilonium or pharmaceutically acceptable salt.

As used herein a unit dose powder is a sachet containing a single dose of a pharmaceutical formulation.

The pharmaceutical formulation of the sachet can be formulated as a powder, granules or pellets or effervescent granules or effervescent pellets or effervescent dry powder. Said powder, granules or pellets or their mixtures may be ready to use sachet's formulation which can be consumed directly from the packet or subsequently after the addition of water.

The powder, granules or pellets of sachet may be 'ready-to-use' formulations, which can be consumed directly from the packet. Alternatively, the powders, granules or pellets or their mixtures of sachet may be formulated for reconstitution with water or another suitable liquid. Powders, granules or pellets of sachet may be in effervescent form. "Granule" refers to particles or granulates or spheronized particles.

The term sachet is used herein to describe any suitable container, package, bag and the like. Preferably the sachet is sealed and disposable. The sachet may be made up from any suitable material, such as plastic, metal foil, paper, and combination thereof. The sachet includes a perforated region or a nick in the edge of the sachet for ease of tearing.

The unit dose sachet may be launched and sold individually or may be launched or sold two or more sachets.

In accordance with this invention, sachet formulation comprises; otilonium or pharmaceutically acceptable salt thereof is in the range of from about 1% to about 40%, diluent is in the range of from about 5 % to about 95%, flavour enhancer is in the range of from 0.1 % to about 15%, sweetener is in the range of from about 0.5% to about 25% , glidant is in the range of from 0.1 % to 15% , flavour is in the range of from about 0.1 % to 15% by the weight of total pharmaceutical composition.

Preferably sachet formulation comprises; otilonium or pharmaceutically acceptable salt thereof is in the range of from about 5% to about 20%, diluent is in the range of from about 30 % to about 80%, flavour enhancer is in the range of from 0.5% to about 8 %, sweetener is in the range of from about 3% to about 20 % , glidant is in the range of from 1.5 % to 6% , flavour is in the range of from about 1.5 % to 7 % by the weight of total pharmaceutical composition.

More preferably sachet formulation comprises; otilonium or pharmaceutically acceptable salt thereof is in the range of from about 10 % to about 15 %, diluent is in the range of from about 60 % to about 70%, flavour enhancer is in the range of from 1 % to about 2 %, sweetener is in the range of from about 4.5 % to about 15 %, glidant is in the range of from 3 % to 4 % , flavour is in the range of from about 3 % to 4 % by the weight of total pharmaceutical composition.

Most preferably, sachet pharmaceutical composition comprising otilonium bromide is 13.33%, diluent is 66.67%, flavour enhancer is 1.66%, sweetener is 11.66%, glidant is 3.34%, and flavour is 3.34% by the weight of total pharmaceutical composition.

In another aspect of this invention, effervescent tablet formulation of otilonium or pharmaceutically acceptable salts thereof is presented.

Although effervescent tablets are generally added to an aqueous medium such as water prior to oral administration, they may be designed to disintegrate in the mouth. Most commonly effervescent tablets are resulting in the formation of a solution or a suspension and the evolution of carbon dioxide gas. Effervescent tablet are making use of a chemical reaction between a soluble acid and an alkali metal carbonate with the help of water to make CO₂ gas, which can give a fizzy taste in the mouth.

Any conventional effervescent agent for example a mixture of a substance which on dissolution in water forms an acid reacting solution, and a pharmaceutically acceptable carbonate can be used as effervescent agent.

The acid component of the composition can be any suitable acid for effervescent compositions. Typically, the acid is organic or mineral acid that is safe and approved for consumption and pharmaceutical and/or nutritional purposes which provides effective and rapid effervescent disintegration upon contact with water and the alkaline effervescent compound. The acid may be selected from food acids, acid anhydrides and acid salts.

The food acids are most commonly used since they are generally regarded as safe (GRAS), they occur in nature and are ingestible. Example of food acid includes are, but not limited to, citric acid, tartaric acid, ascorbic acid, fumaric acid, malic acid, adipic and succinic acid, acetyl salicylic acid, nicotinic acid, mixtures thereof and the like.

Citric anhydride, succinic anhyride, glutamic anhydride can be used as well.

The acid salt of the composition can be any suitable acid salt or any mixture of suitable salts. Examples of such a suitable acid salts or mixture of suitable salts includes sodium hydrogen phosphate, disodium dihydrogen pyrophospate, acid citrate salts, aminoacid hydrochlorides. Combinations thereof are possible.

In effervescent formulations, alkaline excipients can be used. The alkaline component can be any suitable alkaline effervescent compound those are safe and approved for pharmaceutical and/or nutritional purposes, and typically it is an organic base (e.g., an alkali metal carbonate). It provides an effective and rapid effervescent disintegration upon contact with water and the acid compound. The alkaline effervescing compound may be selected from the group consisting of carbonate salts, bicarbonate salts, and mixtures thereof. Example of carbonate sources , but not limited to, sodium bicarbonate, sodium carbonate, potassium bicarbonate and potassium carbonate, sodium sesquicarbonate, sodium glycine carbonate, I-lysine carbonate, arginine carbonate, amorphous calcium carbonate and mixtures thereof.

In accordance with this invention effervescent tablet formulation comprises; otilonium bromide is in the range of from about 1% to about 40%, basic component is in the range of from about 5 % to about 95%, acid component is in the range of from 3 % to about 45%, sweetener is in the range of from about 0.5% to about 25 %, flavour is in the range of from about 0.1 % to 15% by the weight of total pharmaceutical composition.

Preferably effervescent tablet formulation comprises; otilonium bromide is in the range of from about 10% to about 25%, basic component is in the range of from about 20 % to about 60%, acid component is in the range of from 15 % to about 30%, sweetener is in the range of from about 3% to about 25%, flavour is in the range of from about 0.1 % to 10% by the weight of total pharmaceutical composition.

More preferably, effervescent tablet formulation comprises; otilonium bromide is in the range of from about 15% to about 18%, basic component is in the range of from about 35 % to about 50%, acid component is in the range of from 20 % to about 25 %, sweetener is in the range of from about 10% to about 20 %, flavour is in the range of from about 3 % to 5 % by the weight of total pharmaceutical composition.

Most preferably, an effervescent tablet pharmaceutical composition comprising otilonium bromide is 16.00%, basic component is 40.00%, acid component is 24.00%, sweetener is 16.00% and flavour is 4.00% by the weight of total pharmaceutical composition.

In another aspect of this invention, otilonium or pharmaceutically acceptable salts thereof are provided in so called "dry syrup" form to IBS patients. A dry syrup preparation means "preparation with water which is dissolved or suspended before use". The powder is provided in a suitable container such as glass or polyethylene bottle to which prior to use the prescribed amount of water is added to give dispersion. The desired amount of the dispersion is taken orally by the patient according to his need. Such multiple variable "dry syrup" can be particularly used for children.

In a further aspect, the dry syrup formulation is rapidly dispersed in an aqueous fluid and orally administered, for example by means of a spoon or drop by drop.

In accordance with this invention, dry syrup formulation comprises; otilonium bromide is in the range of from about 0.5 % to about 40%, diluent is in the range of from about 5% to about 95%, viscosity increasing agent is in the range of from about 0.01 % to about 5 %, buffering component is in the range of from about 0.01 % to about 5 %, suspending agent is in the range of from 3 % to about 20 %, sweetener is in the range of from about 0.1 % to about 25 %, flavour is in the range of from about 0.1 % to 10% by the weight of total pharmaceutical composition.

Preferably dry syrup formulation comprises; otilonium bromide is in the range of from about 0.5 % to about 10%, diluent is in the range of from about 30 % to about 85 %, viscosity increasing agent is in the range of from about 0.03% to about 3 %, buffering component is in the range of from about 0.02 % to about 3 %, suspending agent is in the range of from 5 % to about 15 %,sweetener is in the range of from about 1 % to about 10 %, flavour is in the range of from about 0.5 % to 5 % by the weight of total pharmaceutical composition.

More preferably dry syrup formulation comprises; otilonium bromide is in the range of from about 1 % to about 5%, diluent is in the range of from about 75 % to about 83 %, viscosity increasing agent is in the range of from about 0.4 % to about 1 %, buffering component is in the range of from about 0.4 % to about 1 %, suspending agent is in the range of from 9 % to about 12 %, sweetener is in the range of from about 1.5 % to about 5 %, flavour is in the range of from about 1 % to 3 % by the weight of total pharmaceutical composition.

Most preferably, dry syrup formulation comprises otilonium bromide is 2.33%, diluent is 80.95%, viscosity increasing agent is 0.67%, buffering agent is 0.88 %, suspending agent is 11.75 %, sweetener is 2.25 %, flavour is 1.17% by the weight of total pharmaceutical composition.

Dry syrup may contain sweeteners. Sugars contained in the dry syrup preparation of the present invention include saccharide and sugar alcohol, or mixtures thereof, as exemplified by at least one kind of compound selected from the group of sucrose, glucose, mannitol, powdered hydrogenated maltose syrup, maltitol, erythritol, sorbitol, maltose, lactose, starch and starch derivative, mannose, sorbose, xylose, trehalose, fructose, dextran, pullulan, dextrin, cyclodextrin, soluble starch, hydroxyethyl starch, sodium carboxymethyl cellulose, inositol, dulcitol, xylitol, arabitol, raffinose, lactitol, etc. Among them, sucrose is preferable. Additionally, the sugars may include natural sweeteners or synthetic sweeteners of non-sugar, such as aspartame, glycyrrhizinic acid and its salt, saccharin and its salt, stevia and its salt, sucralose, and acesulfame potassium and mixture thereof and the like.

Buffering components are added to stabilize the suspension to a desired pH range. Buffering agents are but not limited to, salts of week acids such as carbonates, citrates, gluconates, phosphate and tartrates and mixtures thereof and the like. Citric acid and sodium citrate are mostly used to stabilize pH.

Viscosity increasing agents /suspending agents are, but not limited to, cellulose derivatives such as hydroxypropylcellulose, hydroxypropyl metylcellulose, methylcellulose, hydroxyethylcellulose, carboxymethylcellulose, sodium carboxymethylcellulose, microcrystalline cellulose, acacia, alginates, tragacanth, xanthan gum, bentonite, carbomer, carageenan, powdered cellulose, gelatin and mixtures thereof and the like. Xanthan gum is preferred.

Pharmaceutically acceptable additives other than those described above may be included in sachet, effervescent, dry syrup formulations of the present invention of otilonium or pharmaceutically acceptable salts. The present invention's formulation comprises other ingredients, including pharmaceutically acceptable diluents, flavour enhancers, preservatives, flavourings, colouring agents, binders, disintegrants, lubricants, glidants.

Diluents are, but not limited to, anhydrous lactose, lactose monohydrate, modified lactose, dibasic calcium phosphate, tribasic calcium phosphate, microcrystalline cellulose, silicified microcrystalline cellulose, powdered cellulose, maize starch, pregelatinized starch, calcium carbonate, sucrose, glucose, dextrates, dextrins, dextrose, fructose, lactitol, mannitol, sorbitol, starch, mixtures thereof and the like.

Flavour enhancers are, but not limited to, citronellyl acetate, geranyl acetate, geranyl propionate, linalool, alpha-ionone, 2,2,6-trimethyl-6-vinyl-tetra-hydropyran, citronella oil, mixtures thereof and the like.

Preservatives are added to prevent microbial growth. Preservatives are, but not limited to, propylene glycol, disodium edentate, Benzalkonium chloride, benzoic acid, cetrimide, chlorobutanol, phenyl mercuric acetate, potassium sorbate, sodium benzoate, sorbic acid, parabens such as methyl paraben ethyl, propyl or butyl paraben or pharmaceutically acceptable salts and mixtures thereof and the like.

Flavourings are, but not limited to, cinnamon oil, essence of apple, essence of pear, essence of peach, essence of grape, essence of strawberry, essence of raspberry, essence of cherry, essence of plum, essence of pineapple, essence of apricot, oil of wintergreen, peppermint oils, clove oil, bay oil, anise oil, eucalyptus, thyme oil, cedar leaf oil, oil of nutmeg, oil of sage, oil of bitter almonds, cassia oil, citrus oils such as lemon, orange, grape, lime and grapefruit , vanilla, benzaldehyde , aldehyde C-8 , aldehyde C-9, aldehyde C-12 , tolyl aldehydeacacia, anise oil, benzaldehyde, caraway oil, cardamom (oil, tincture, spirit),cherry syrup, citric acid syrup, citric acid, clove oil, cocoa, cocoa syrup, coriander oil, dextrose, ethyl acetate, ethyl vanillin, fennel oil, ginger, glucose, glycerin, glycerrhiza, honey, lavender oil, lemon oil, mannitol, nutmeg oil, methyl salicylate, orange oil, orange flower water, peppermint (oil, spirit, water), raspberry, rose (oil, water), rosemary oil, saccharin sodium, Sarsaparilla syrup, spearmint oil, thyme oil, tolu balsam, vanilla ,vanilla tincture, tolu balsam syrup, wild cherry syrup and mixtures thereof and the like.

Colouring agents are added to impart desired color and improve elegance. Colourings agents are but not limited to, titanium dioxide, brilliant blue, indigo carmine, ammaranth, tartarazine, sunset yellow, carmine, caramel, chlorophyll, annatto seeds, carrots, madder plant, indigo saffron and mixtures thereof and the like.

Binders are, but not limited to, polyvinylpyrolidone, copovidone, starches such as pregelatinized starch or plain starch, cellulose derivatives such as hydroxypropylmethylcellulose, ethylcellulose, hydroxypropylcellulose and carboxymethylcellulose and their salts, gelatine, acacia, agar, alginic acid,carbomer, ceratonia, chitosan, dextrates,dextrin,glycerol dibehenate, guar gum, hypromellose, inulin, magnesium aluminumsilicate, maltodextrin, poloxamer, polycarbophil, polydextrose, polyethylene oxide, polymethacrylates, sodium alginate, sucrose, hydrogenated vegetable oil, mixtures thereof and the like.

Disintegrants are , but not limited to, modified starches, croscarmallose sodium, carboxymethylcellulose calcium, sodium starch glycolate, crospovidone, alginic acid, calcium alginate, microcrystalline cellulose, powdered cellulose, chitosan, colloidal silicon dioxide, crospovidone, guar gum, low-substituted hydroxypropylcellulose, hydroxypropyl starch, magnesium aluminium silicate, methylcellulose, polacrilin potassium, sodium alginate, starch, pregelatinized starch, mixtures thereof and the like.

Lubricants are, but not limited to, magnesium stearate, calcium stearate, hydrogenated castor oil, glyceryl behenate, glyceryl monostearate, glyceryl palmitostearate, leucine, mineral oil, light mineral oil, myristic acid, palmiticacid, polyethylene glycol, potassium benzoate, sodium benzoate, sodium lauryl sulfate, sodium stearyl fumarate, stearic acid, talc, hydrogenated vegetable oil, zinc stearate, magnesium lauryl sulphate, sodium stearyl fumarate, polyethylene glycol, stearic acid, colloidal silicon dioxide or mixtures thereof and the like.

The compositions of the present invention preferably also contain a glidant. Glidants are preferably selected from the group consisting of colloidal silicon dioxide, precipitated silica and pyrogenic silica, talc and aluminium silicate, tribasic calcium phosphate, calcium stearate, powdered cellulose, magnesium oxide, magnesium silicate, magnesium trisilicate, starch, talc, mixtures thereof and the like. Colloidal silicon dioxide is preferred.

The compositions may include antiadherants, surfactants, solubilizers, wetting agents and other excipients of common use. Additives ingredients will vary according to the type of compositions being manufactured and can be included in the pharmaceutical composition in any amount.

The dry syrup preparation of the present invention is produced using a method for producing a preparation of powders, granules, or fine granules, as exemplified by mixing-granulating method, extrusion-granulating method, fluidized bed-granulating method, chopping granulating method, spray-granulating method, and crushing granulating method, one-pot processing method.

Formulations and preparing methods are illustrated by the following examples without being limited but not intended to limit the scope of the invention.

### Example 1 : Sachet Formulation and Preparing Methods Thereof

**Table 1**

| **SACHET FORMULATION** | | |
|---|---|---|
| **Ingredients** | **mg** | **%** |
| Otilonium Bromide | 40.00 | 13.33 |
| Maltodextrin | 200.00 | 66.67 |
| Citric Acid | 5.00 | 1.66 |
| Sucralose | 20.00 | 6.66 |
| Acesulfame | 15.00 | 5.00 |
| Potassium | | |
| Colloidal Silicon | 10.00 | 3.34 |
| Dioxide | | |
| Lemon Flavour | 10.00 | 3.34 |
| **Total** | **300.00** | **100.00** |

Formulation above can be prepared by means of a preparation method comprising steps of;

a. Otilonium bromide, citric acid, sucralose, acesulfame potassium and colloidal silicon dioxide are mixed in a container.

b. The powder mixture is sieved by using a suitable screen and installed into container and mixed.

c. The powder prepared at step (b) is added to a portion of maltodextrin.

d. The rest of maltodextrin is added to the container and mixed.

e. Average target powder weight is filled out to sachets.

Formulation above can be prepared by means of a wet granulation method comprising intra granulation and extra granulation steps as following manner;

**Intragranulation**:

a. Otilonium bromide, citric acid, sucralose, acesulfame potassium are dissolved in water.

b. The granulation solution prepared at step (a) is added/ sprayed onto maltodextrin in the granulator to form wet granules.

c. Wet granules are mixed and transferred to a fluidized bed dryer until proper moisture then sizing the dried granules by using suitable milling equipment.

**Extragranulation**:

d. Flavour and glidant are mixed and sieved by using suitable milling equipment.

e. Dried granules and the blend prepared at step d are mixed.

f. Appropriate drug load is filled out to sachets and sachet is sealed.

### Example 2 : Effervescent Formulation and Preparing Methods Thereof

**Table 2**

| **EFFERVESCENT FORMULATIONS** | | |
|---|---|---|
| **Ingredients** | **mg** | **%** |
| Otilonium Bromide | 40.00 | 16.00 |
| Sodium Bicarbonate | 100.00 | 40.00 |
| Citric Acid | 60.00 | 24.00 |
| Sucralose | 25.00 | 10.00 |
| Sodium Saccharin | 15.00 | 6.00 |
| Orange Flavour | 10.00 | 4.00 |
| **Total** | **250.00** | **100.00** |

Effervescent tablet or effervescent powder or effervescent granules can be prepared by the following method comprising steps of;

a. Otilonium bromide, sucralose and sodium saccharin, orange flavours are mixed in a container.

b. The powder mixture is sieved by using a suitable screen and installed into container and mixed.

c. The powder prepared at step (b), sodium bicarbonate and citric acid are added to the container and mixed.

d. The rest of diluents is added to the container, mixed and sieved.

e. The final mixture is compressed by using appropriate punches or

Effervescent tablet or effervescent powder or effervescent granules can also be prepared by the following method:

1. Sodium bicarbonate, citric acid, orange flavour are mixed and sieved by using a screen.

2. Otilonium bromide, sucralose, sodium saccharin are dissolved in water.

3. The powder mixture prepared in step (1) is granulated with solution prepared in Step (2) utilizing granulator to form wet granules.

4. Wet granules are mixed and transferred to a fluidized bed dryer until proper moisture then sizing the dried granules by using suitable milling equipment.

The final mixture is compressed by using appropriate punches.

### Example 3 : Dry Syrup Formulation and Preparing Methods Thereof

**Table 3**

| **DRY SYRUP FORMULATION** | | |
|---|---|---|
| **Ingredients** | **Mg/bottle** | **%** |
| Otilonium Bromide | 280.00 | 2.33 |
| Xanthan Gum | 80.00 | 0.67 |
| Citric Acid | 30.00 | 0.25 |
| Sodium Citrate | 75.00 | 0.63 |
| Sucralose | 160.00 | 1.33 |
| Acesulfame Potassium | 110.00 | 0.92 |
| Colloidal Silicon Dioxide | 85.00 | 0.71 |
| Carmellose Sodium | 1,325.00 | 11.04 |
| Mannitol | 9,715.00 | 80.95 |
| Cherry Flavour | 140.00 | 1.17 |
| Total Weight in Bottle | 12,000.00 | 100.0 |

Dry syrup formulation can be prepared a manufacturing method comprises steps of:
1. Xanthan gum, citric acid, sodium citrate, sucralose, acesulfame Potassium, colloidal silicon dioxide and cherry flavour are mixed.
2. Powder mixture in Step 1 is optionally sieved through a suitable sieve and mixed again.
3. Otilonium bromide and carmellose sodium are added to powder mixture in step 2 and mixed.
4. A portion of mannitol and powder mixture in step 3 and the rest of mannitol are added and mixed.
5. Powder mixture prepared in step 4 is filled into suitable packaging material, such as type III glass bottles or HDPE bottles, having average target powder weight of 12000 mg/bottle.

## Claims

1. A unit dose of sachet containing pharmaceutical formulation comprising otilonium or pharmaceutically acceptable salt thereof and an excipient or mixtures of excipients.

2. Sachet, as claimed in claim 1, includes dry powder or granules or pellets or effervescent granules or effervescent pellets or effervescent dry powder or mixtures thereof.

3. Dry powder or granules or pellets of otilonium, as claimed in claim 2, show a feature to be suspended or dispersed or dissolved in a solution.

4. Dry powder or granules or pellets of otilonium as claimed in claim 2 is directly administered without water or liquid.

5. The unit dose of sachet, as claimed in claim 1, comprises; A-) otilonium or pharmaceutically acceptable salt thereof is in the range of from about 1% to about 40%, diluent is in the range of from about 5 % to about 95%, flavour enhancer is in the range of from 0.1 % to about 15%, sweetener is in the range of from about 0.5% to about 25% , glidant is in the range of from 0.1 % to 15%, flavour is in the range of from about 0.1 % to 15% by the weight of total pharmaceutical composition or B-) preferably otilonium or pharmaceutically acceptable salt thereof is in the range of from about 5% to about 20%, diluent is in the range of from about 30 % to about 80%, flavour enhancer is in the range of from 0.5% to about 8 %, sweetener is in the range of from about 3% to about 20 % , glidant is in the range of from 1.5 % to 6% , flavour is in the range of from about 1.5 % to 7 % by the weight of total pharmaceutical composition or C-) more preferably otilonium or pharmaceutically acceptable salt thereof is in the range of from about 10 % to about 15 %, diluent is in the range of from about 60 % to about 70%, flavour enhancer is in the range of from 1 % to about 2 %, sweetener is in the range of from about 4.5 % to about 15 % , glidant is in the range of from 3 % to 4 % , flavour is in the range of from about 3 % to 4 % by the weight of total pharmaceutical composition or D-) most preferably otilonium bromide is 13.33%, diluent is 66.67%, flavour enhancer is 1.66%, sweetener is 11.66%, glidant is 3.34%, and flavour is 3.34% by the weight of total pharmaceutical composition.

6. An effervescent tablet comprising otilonium or pharmaceutically acceptable salt thereof and an excipient or mixtures of excipients.

7. Effervescent tablet, as claimed in claim 6, show a feature to be suspended or dispersed or dissolved in a solution.

8. Effervescent tablet, as claimed in claim 7, is directly administered without water or liquid.

9. The effervescent tablet, as claimed in claim 6, comprises; **A-)** otilonium bromide is in the range of from about 1 % to about 40%, basic component is in the range of from about 5 % to about 95%, acid component is in the range of from 3 % to about 45%, sweetener is in the range of from about 0.5% to about 25 %, flavour is in the range of from about 0.1 % to 15% by the weight of total pharmaceutical composition **or B-)** preferably otilonium bromide is in the range of from about 10% to about 25%, basic component is in the range of from about 20 % to about 60%, acid component is in the range of from 15 % to about 30%, sweetener is in the range of from about 3% to about 25 % , flavour is in the range of from about 0.1 % to 10% by the weight of total pharmaceutical composition **or C-)** more preferably otilonium bromide is in the range of from about 15% to about 18%, basic component is in the range of from about 35 % to about 50%, acid component is in the range of from 20 % to about 25 %, sweetener is in the range of from about 10% to about 20 % , flavour is in the range of from about 3 % to 5 % by the weight of total pharmaceutical composition **or D-)** most preferably otilonium bromide is 16.00%, basic component is 40.00%, acid component is 24.00%, sweetener is 16.00% and flavour is 4.00% by the weight of total pharmaceutical composition.

10. A dry syrup comprising otilonium or pharmaceutically acceptable salts thereof and an excipient or mixtures of excipients.

11. Dry syrup form, as claimed in claim 10, comprises dry powder or granules or pellets or mixtures thereof.

12. Particles of otilonium as claimed in claim 11 show a feature to be suspended or dispersed or dissolved in a solution.

13. Dry syrup, as claimed in claim 10, comprises; **A-)** otilonium bromide is in the range of from about 0.5 % to about 40%, diluents is in the range of from about 5% to about 95%, viscosity increasing agent is in the range of from about 0.01 % to about 5 %, buffering component is in the range of from about 0.01 % to about 5 %, suspending agent is in the range of from 3 % to about 20 %, sweetener is in the range of from about 0.1 % to about 25 %, flavour is in the range of from about 0.1 % to 10% by the weight of total pharmaceutical composition **or B-)** preferably otilonium bromide is in the range of from about 0.5 % to about 10%, diluents is in the range of from about 30 % to about 85 %, viscosity increasing agent is in the range of from about 0.03% to about 3 %, buffering component is in the range of from about 0.02 % to about 3 %, suspending agent is in the range of from 5 % to about 15 %, sweetener is in the range of from about 1 % to about 10 %, flavour is in the range of from about 0.5 % to 5 % by the weight of total pharmaceutical composition **or C-)** more preferably otilonium bromide is in the range of from about 1 % to about 5%, diluents is in the range of from about 75 % to about 83 %, viscosity increasing agent is in the range of from about 0.4 % to about 1 %, buffering component is in the range of from about 0.4 % to about 1 %, suspending agent is in the range of from 9 % to about 12 %, sweetener is in the range of from about 1.5 % to about 5 %, flavour is in the range of from about 1 % to 3 % by the weight of total pharmaceutical composition **or D-)** most preferably otilonium bromide is 2.33%, diluent is 80.95%, viscosity increasing agent is 0.67%, buffering agent is 0.88 %, suspending agent is 11.75 %, sweetener is 2.25 %, flavour is 1.17% by the weight of total pharmaceutical composition.
